# EUROPEAN PATENT SPECIFICATION

(11) **EP 3 326 543 B1**
(45) Date of publication and mention of the grant of the patent: **24.07.2019**
(21) Application number: 17194609.8
(22) Date of filing: 24.08.2005
(51) Int. Cl.: A61B 17/04, A61B 17/06

(54) **EXPANDABLE NEEDLE SUTURE APPARATUS AND ASSOCIATED HANDLE ASSEMBLY WITH ROTATIONAL SUTURE MANIPULATION SYSTEM**
WUNDNAHTVORRICHTUNG MIT EXPANDIERBARER NADEL UND ZUGEHÖRIGE GRIFFANORDNUNG MIT DREHENDEM NAHTMANIPULATIONSSYSTEM
APPAREIL DE SUTURE À AIGUILLE EXTENSIBLE ET ENSEMBLE POIGNÉE ASSOCIÉ AVEC SYSTÈME DE MANIPULATION DE SUTURE ROTATIF

(30) Priority: 24.08.2004 US 604243 P
(43) Date of publication of application: 30.05.2018
(62) Divisional of application: 05788810.9
(73) Proprietor: DePuy Mitek, LLC, Raynham, MA 02767 (US)
(72) Inventor: WEISEL, Thomas, Ventura, CA California 93003 (US); PADGET, Martin, Latrobe, PA Pennsylvania 15650 (US)
(74) Representative: Carpmaels & Ransford LLP

(56) References cited:
- EP-A1- 1 402 824
- US-A- 5 312 422
- US-A- 5 618 290
- US-A1- 2002 147 456

## Description

### BACKGROUND OF THE INVENTION

### Field of the Invention

This invention relates generally to surgical suture apparatus.

### Discussion of Related Art

One of the more challenging aspects of endoscopy/arthroscopy is to properly place a suture in identified tissue. This task is complicated by the fact that all work must be done through a 5-8 millimeter cannula while viewing one's efforts on a monitor.

With these difficult size configurations, it has been a significant challenge to incorporate all of the tasks into a single instrument. As a result, the tasks for initially passing a suture through target tissue and subsequently retrieving the suture from the far side has often required use of more than one instrument, especially when the suture is positioned in an awkward or difficult to reach orientation relative to the instrument being used to retrieve the suture. Various needle configurations have been contemplated, some with the needle permanently attached to the suture and others wherein the needle merely engages the suture. Attempts to miniaturize the needle structure have often produced needles that were so small they tended to yield under compressive stresses.

The handles associated with suturing devices have been limited in the number of functions that could be performed. In some cases, handle assemblies have required two-handed operation. It has also been typical to provide needle assemblies that were permanently attached to the handle rather than assemblies that were removable from the handle and perhaps disposable. US5618290A describes an endoscopic suturing device that comprises a needle shaft with an enlarged pointed head at its distal end. The underside of the pointed head at its junction with the shaft forms a shoulder which provides a hook. A latch that is spring-biased to the open position is provided for opening and closing the hook. A tubular sheath surrounds and is concentric with the needle shaft and is dimensioned so that the needle shaft slides within the tubular sheath. The hook can be opened by extending the needle shaft from the distal end of the tubular sheath and closed by retracting the needle shaft. In this way, a suture can be selectively captured or released. A handle at the proximal end includes a grip on the shaft and a cooperating grip on the sheath. The shaft grip can be in the form of a plunger thumb rest, and the sheath grip in the form of a pair of finger rests. Alternatively, the shaft grip is a thumb loop and the sheath grip has finger loops to extend the latch relative the tubular sheath. Rotation of the shaft within the sheath is allowed to vary the presentation of the hook and facilitate manipulation of the suture. The thumb plunger may be rotated to rotate the shaft within the sheath, or a thumbwheel is provided to serve as a rotator for the shaft. Rotation of the shaft at the proximal end affords rotation of the hook at the distal end.

### SUMMARY OF THE INVENTION

The present invention provides a surgical suturing device, as defined in appended claim 1, including a needle assembly having a needle movable in a needle housing between a free suture state and a captured suture state, a handle assembly including a longitudinal handle housing sized and configured to releasably receive the needle assembly, and a thumb actuator assembly releasably coupled to the needle and movable longitudinally on the handle housing between a distal position and a proximal position and between a first lateral position and a second lateral position. The distal position of the thumb actuator assembly is associated with the needle in the free suture state. The proximal position of the thumb actuator assembly is associated with the needle in the captured suture state. The first lateral position of the thumb actuator assembly is associated with the needle in a first angular position. The second lateral position of the thumb actuator assembly is associated with the needle in a second angular position. The thumb actuator assembly comprises a thumb lever that moves axially along the surgical suturing device's axis and that moves side-to-side about the surgical suturing device's axis.

Preferred embodiments of the invention are defined in the appended dependent claims.

### DESCRIPTION OF THE DRAWINGS

Fig. 1 is an axial cross section view illustrating a bifurcated needle assembly;
Fig. 2 is an axial cross section view showing the needle assembly in a retracted position;
Fig. 3 is an enlarged view of the distal end of the device illustrated in Fig 2;
Fig.4 - Fig. 9 illustrate steps in an exemplary method for using the suture device of Fig. 1;

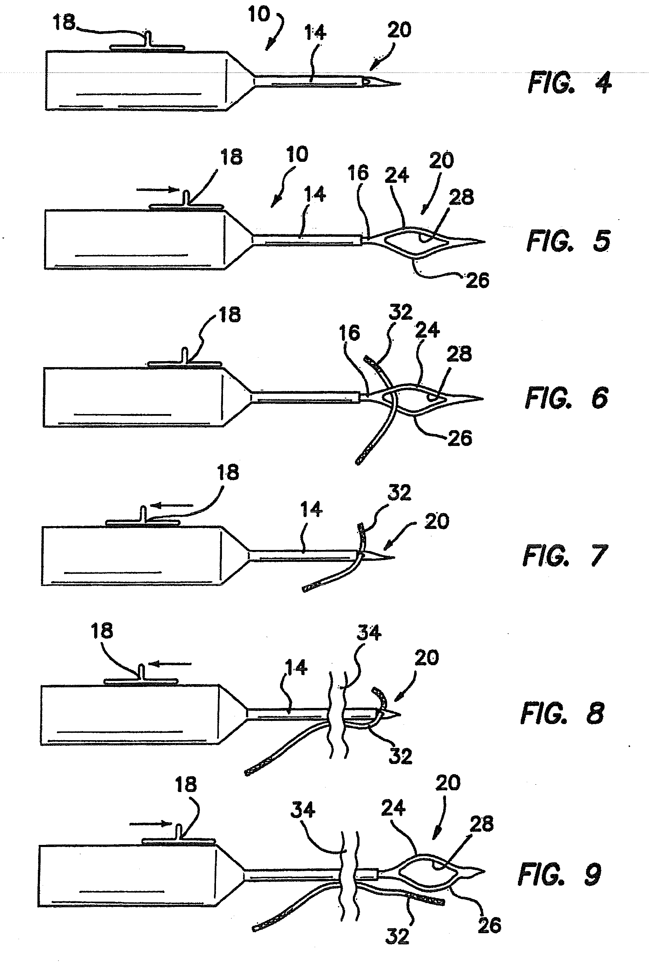

Fig. 4 is a side elevation view illustrating the device in a normal state with the needle assembly retracted;
Fig. 5 is a side elevation view showing operation of a thumb slide to deploy the needle assembly to an extended position characterized by automatic formation of an enlarged slot in the needle assembly;
Fig. 6 is a side elevation view illustrating the loading of a suture into the slot of the needle assembly;
Fig. 7 is a side elevation view showing retraction of the needle assembly and capture of the loaded suture;
Fig. 8 is the side elevation view illustrating passage of the needle assembly and suture through tissue;
Fig. 9 is a side elevation view illustrating deployment of the needle assembly to the extended position to enlarge the slot and release the suture prior to withdrawal of the device from the tissue;

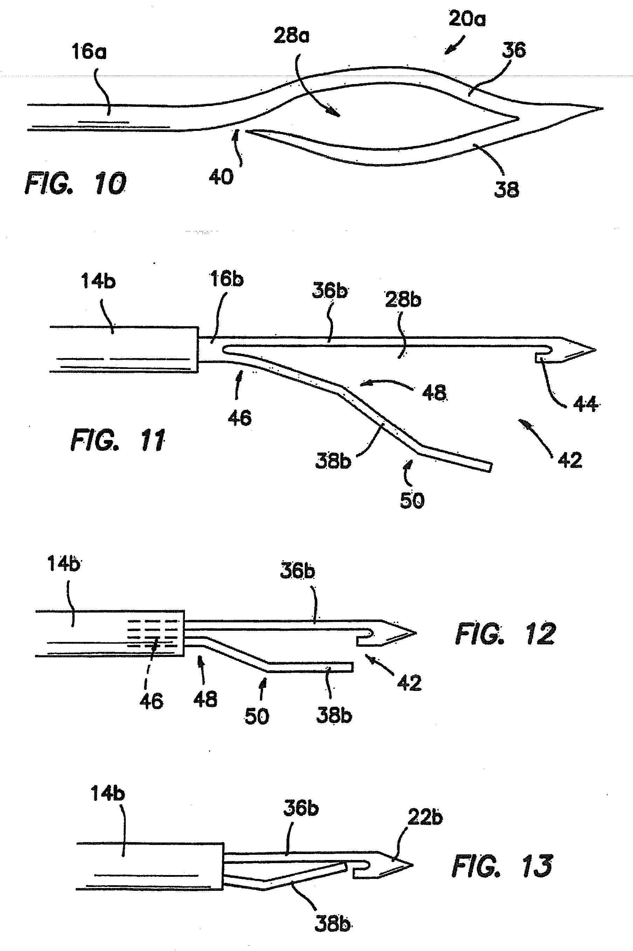

Fig. 10 is a side elevation view wherein the needle is fabricated at the proximal end of the slot;
Figs. 11 -14 illustrate the needle bifurcated at the distal end of the slot;
Fig. 11 is a side elevation view showing a backing arm and a gathering arm widely separated to receive a suture;
Fig. 12 is a side elevation view illustrating the needle slightly retracted;
Fig. 13 is a side elevation view showing the needle further retracted;

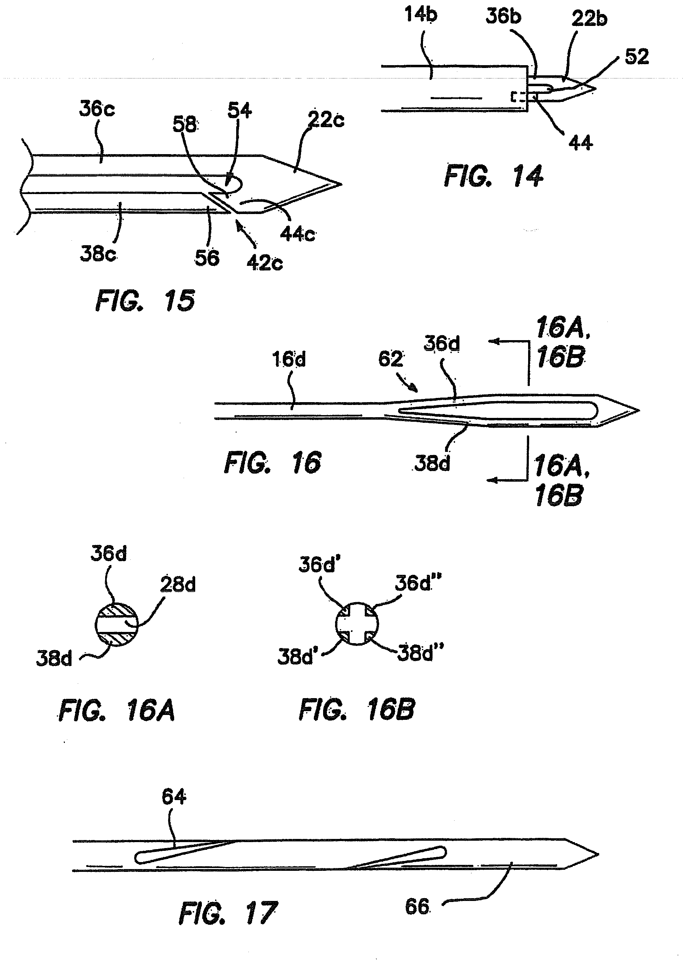

Fig. 14 is a side elevation view showing the needle fully retracted;
Fig. 15 is a side elevation view similar to Figure 11, but including a built-in stop;
Fig. 16 is a side elevation view wherein the needle is tapered to facilitate manipulation through drastically bent tubes;
Fig. 16A is a cross section view taken along lines 16A - 16A of Figure 16 showing two arms;
Fig. 16B is a cross section view taken along lines 16B - 16B of Figure 16 showing four arms;
Fig. 17 is a side elevation view wherein a helical slot is cut around the circumference of the needle;

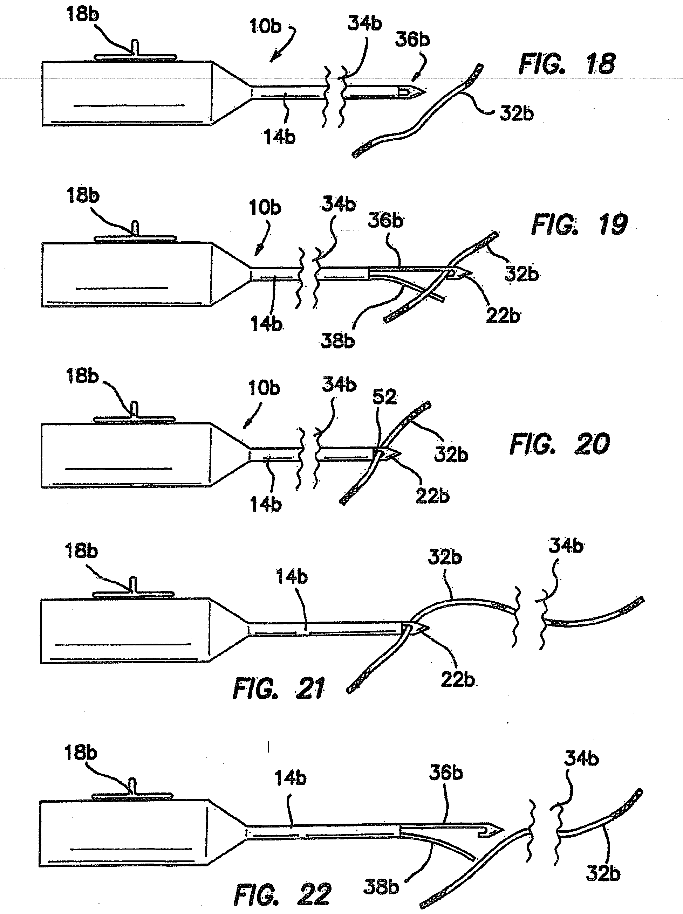

Figs. 18 - 22 illustrate an exemplary method particularly applicable to Figure 11;
Fig. 18 is a side elevation view showing the needle retracted and passed through the tissue;
Fig. 19 is a side elevation view showing the needle fully deployed to receive the suture;
Fig. 20 is a side elevation view showing the needle retracted with the suture disposed in the needle slot;
Fig. 21 is a side elevation view showing the needle removed from the tissue drawing the suture through the needle hole in the tissue;
Fig. 22 is a side elevation view showing the needle fully deployed to release the suture from the needle leaving the suture extending through the tissue;

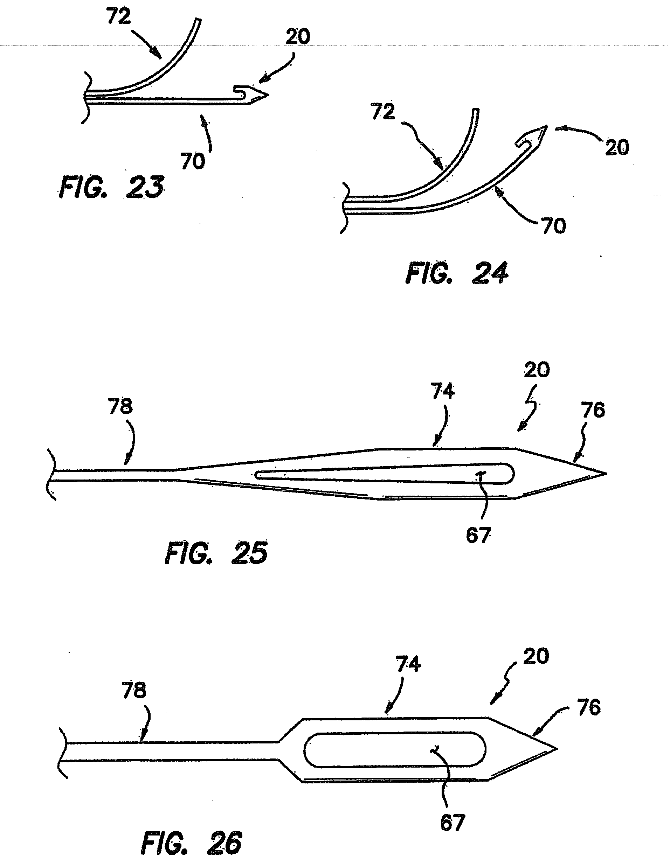

Fig. 23 is a side elevation view of a needle assembly including a straight backing arm and a curved gathering arm;
Fig. 24 is a side elevation view including a curved gathering arm and a curved backing arm;
Fig. 25 is a needle assembly with angled portions defining a slot;
Fig. 26 is a needle assembly with large and small diameter portions;

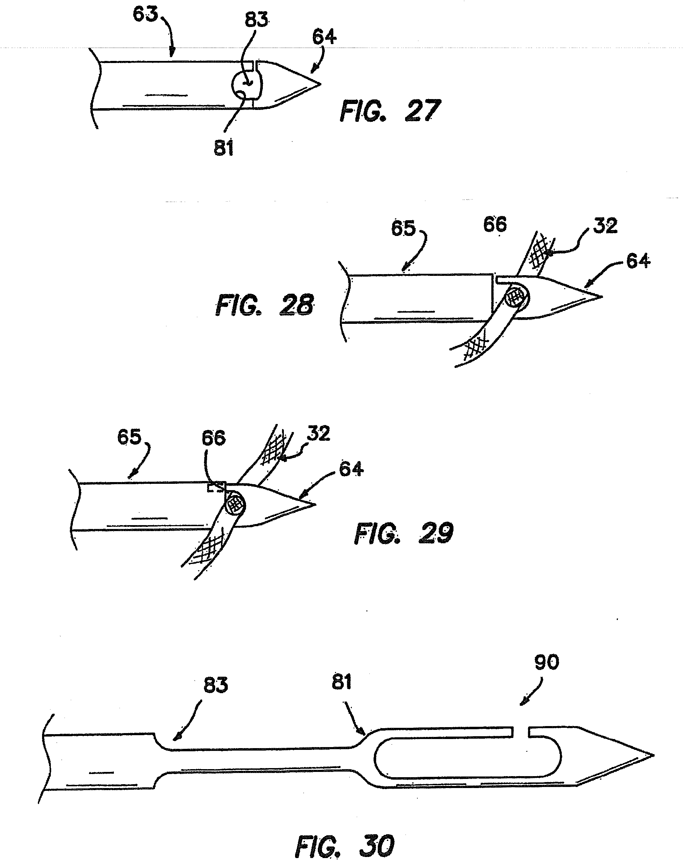

Fig. 27 is a side elevation view of another needle assembly including a notch in the needle tube;
Fig. 28 is a side elevation view illustrating a notch-free needle tube and a needle in a suture-sliding position;
Fig. 29 is a side elevation view similar to Fig. 28 with the needle in a suture-locked position;
Fig. 30 is a side elevation view of a needle assembly having a bendable section;

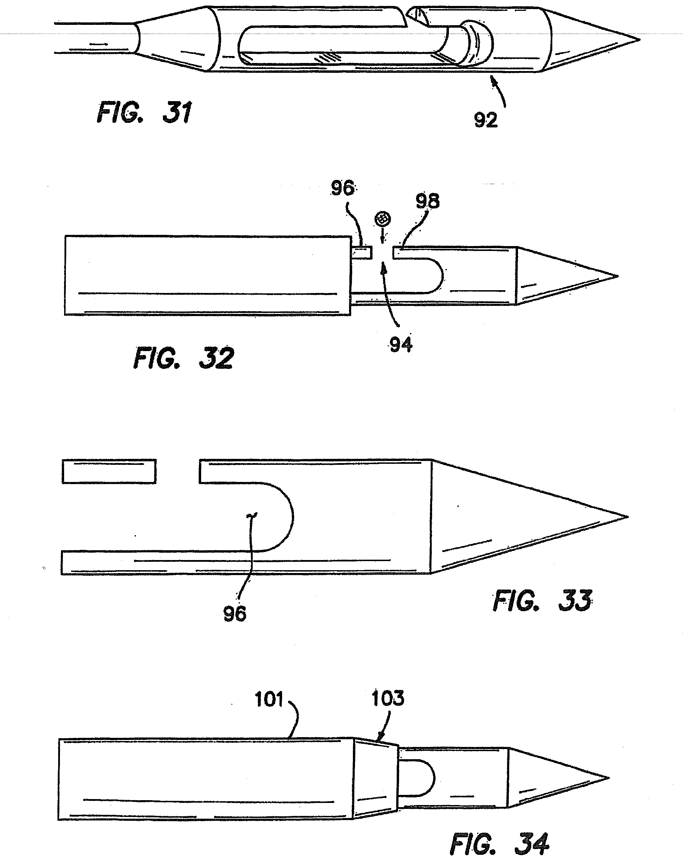

Fig. 31 is a perspective view of the needle assembly having a radius to prevent suture fraying;
Fig. 32 is a side elevation view of a needle assembly having an opening into the suture slot;
Fig. 33 is a side elevation view illustrating the preferred disposition of the opening;
Fig. 34 is a side elevation view illustrating a taper in the needle housing;

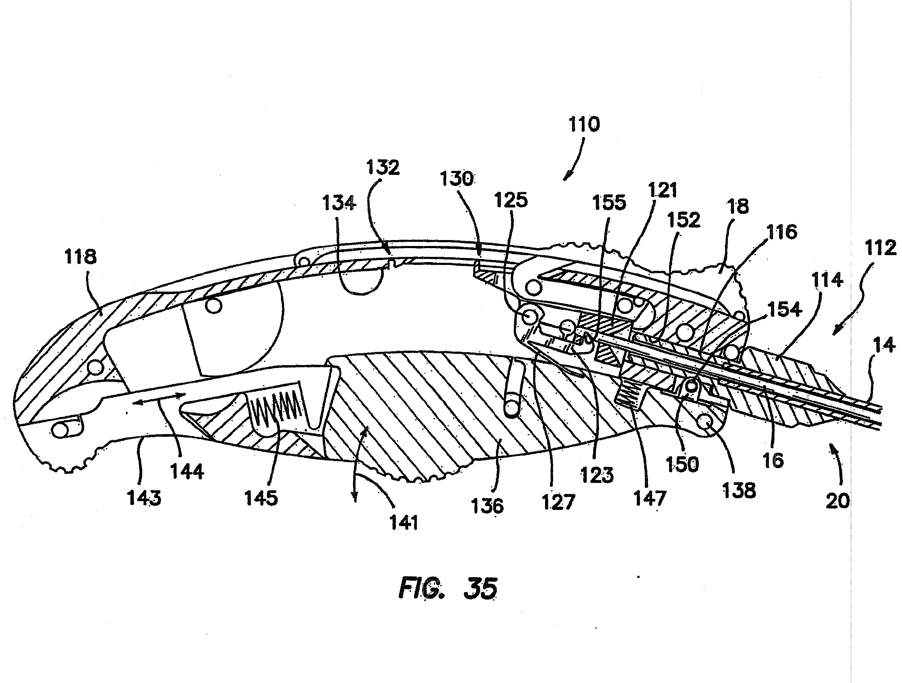

Fig. 35 is a cross-section view of a handle assembly;

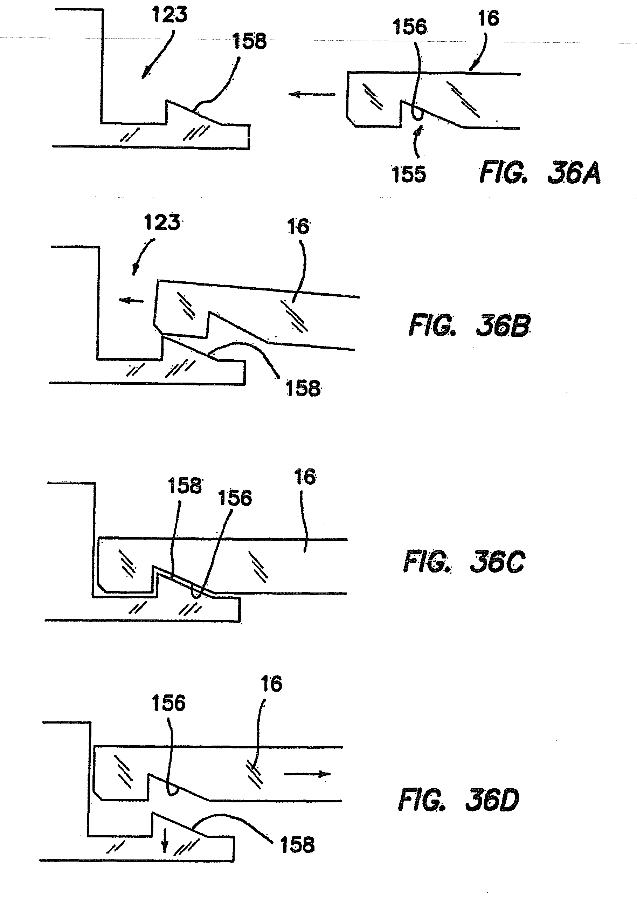

Figs. 36a-36d are progressive views of an exemplary structure and method for coupling the needle assembly and the handle assembly;

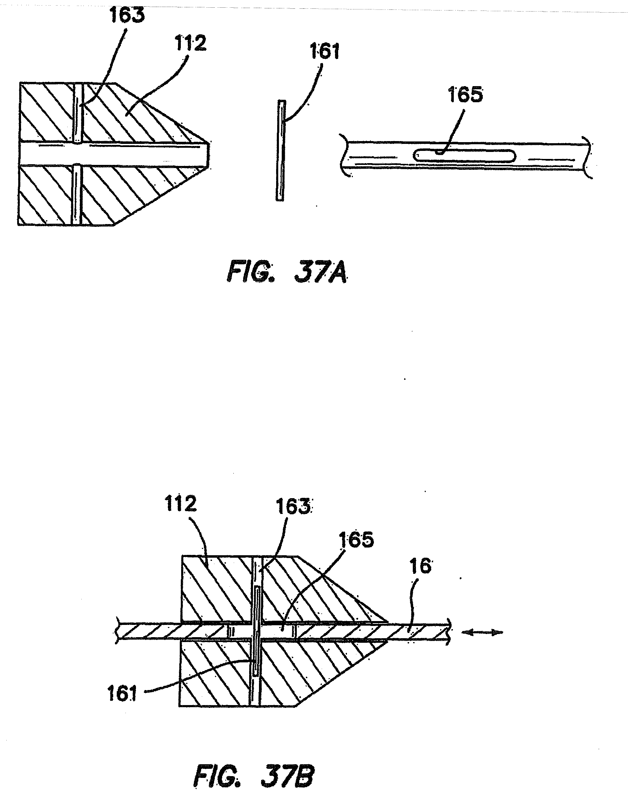

Fig. 37a and 37b are side elevation views illustrating use of a pin to restrict movement of a needle rod;

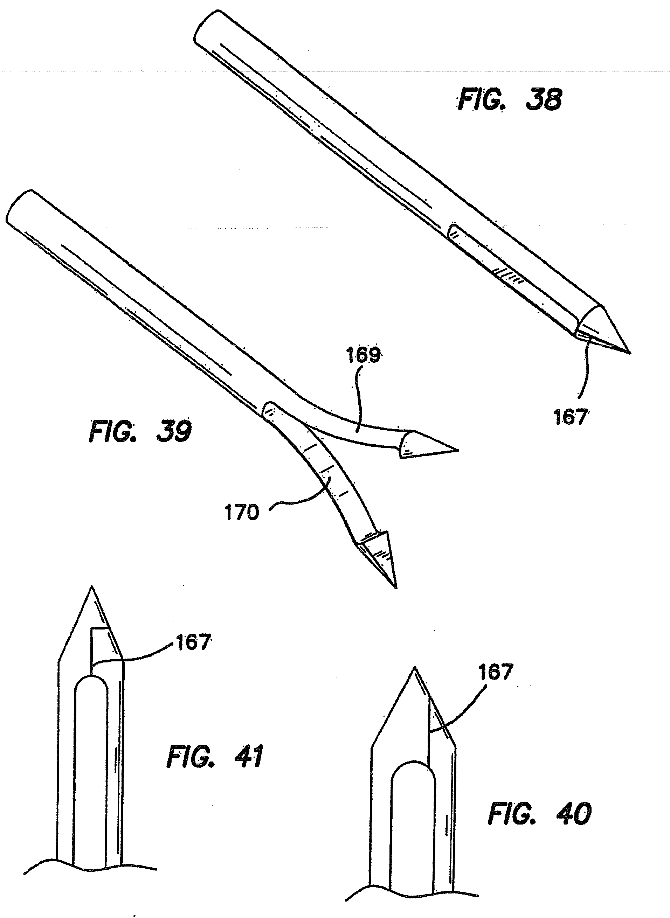

Fig. 38 is a perspective view of a needle assembly bifurcated by a slot;
Fig. 39 is a perspective view illustrating the needle of Figure 38 in its bifurcated configuration;
Fig. 40 is a side elevation view of an additional bifurcated needle assembly;
Fig. 41 is a side elevation view of a further bifurcated needle assembly;

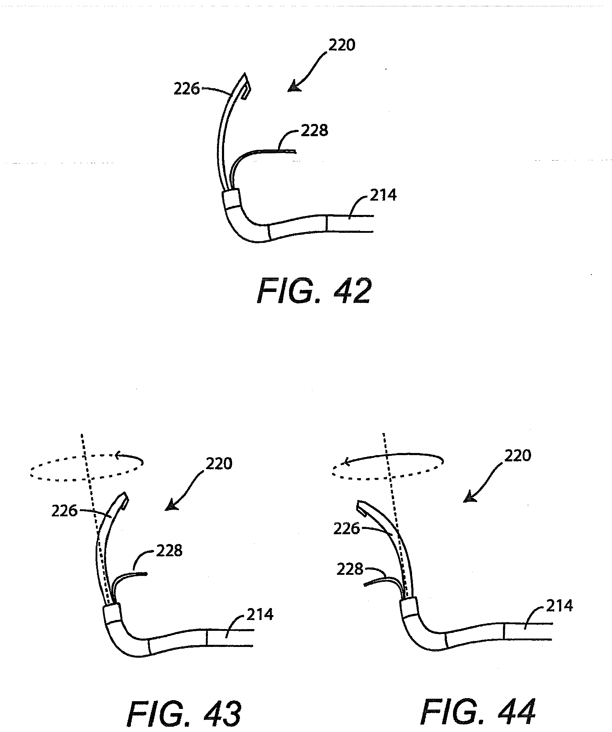

Figs. 42-44 show the distal end of further possible suture devices that can retract and deploy the needle assembly and can rotate the needle assembly to different desired orientations after deployment to aid in the grasping of the suture;
Fig. 42 is a perspective view of the needle assembly in a standard straight position;
Fig. 43 is a perspective view of the needle assembly after being rotated about 45°;
Fig. 44 is a perspective view of the needle assembly after being rotated about 135°;

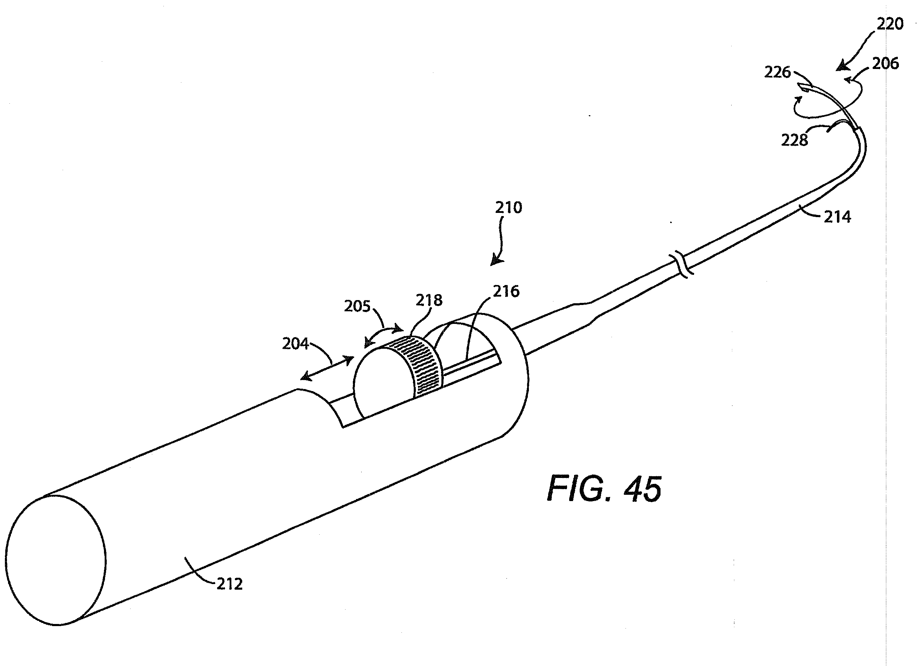

Fig. 45 is a perspective view of a suture device that uses a thumb wheel to deploy and rotate the needle assembly as shown in Figures 42-44;

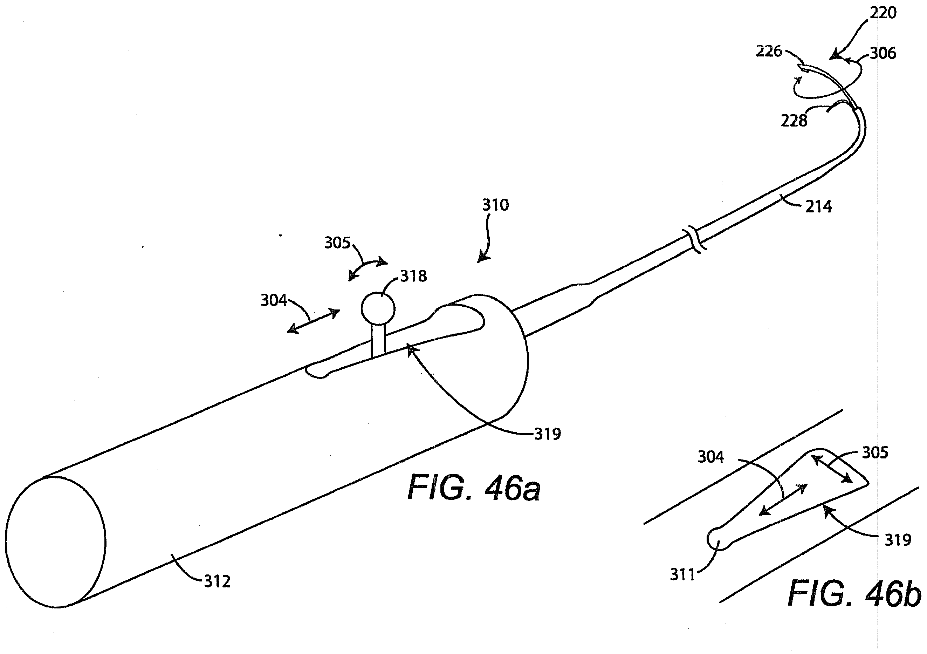

Fig. 46a is a perspective view of a suture device that uses a thumb lever located within a lever channel to deploy and rotate the needle assembly as shown in Figures 42-44;
Fig. 46b is a closeup view of the lever channel of the suture device shown in Figure 46a;

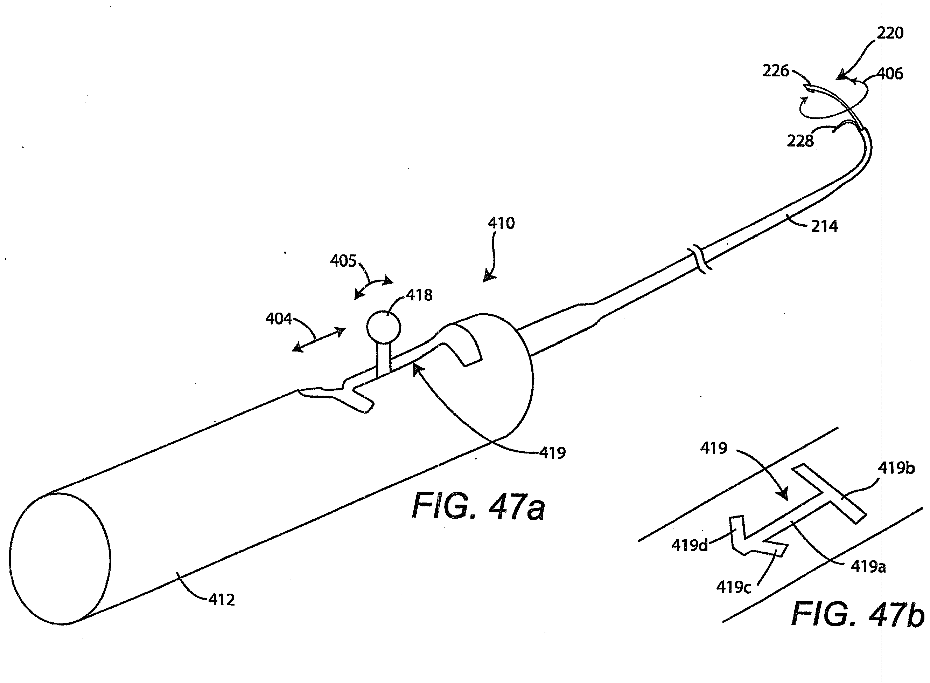

Fig. 47a is a perspective view of a suture device that uses a thumb lever located within a movement-constraining lever channel to deploy and rotate the needle assembly as shown in Figures 42-44;
Fig. 47b is a closeup view of the lever channel of the suture device shown in Figure 47a.

### DETAILED DESCRIPTION

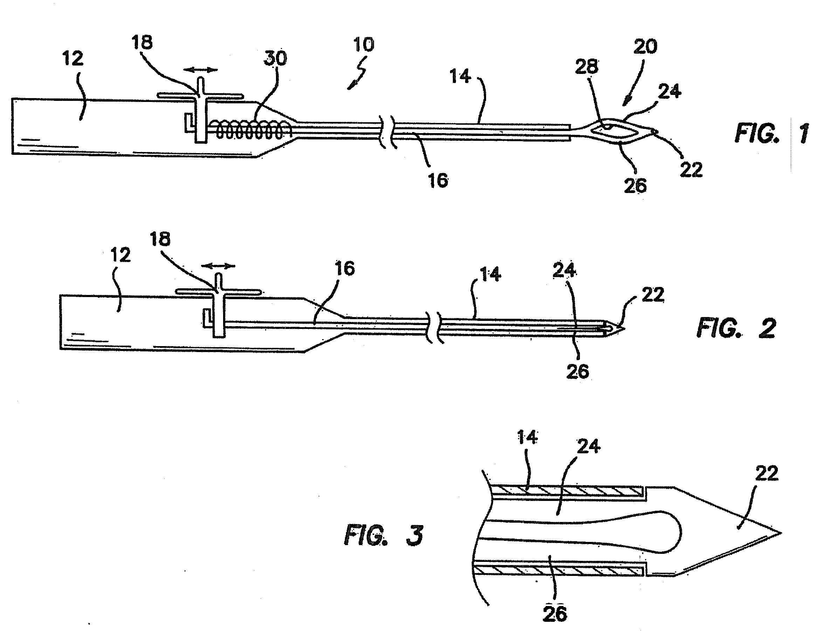

A suture device is illustrated in Figure 1 and designated by the reference numeral 10. The device is an elongate structure and includes a handle 12 at its proximal end and a hollow shaft 14 at its distal end. An actuating rod 16 is disposed within the hollow shaft 14 and extends from the handle 12 through the shaft 14. In the handle 12, the actuating rod 16 is engaged by a thumb slide 18. At its distal end, the actuating rod 16 is coupled to a needle assembly 20. This assembly 20 includes a sharp tip 22 and bifurcated portions 24 and 26, which define a slit or slot 28 proximately of the tip 22. Operation of the thumb slide 18 relative to the handle 12 moves the actuating rod 16 and the attached needle assembly 20 between an extended position as illustrated in Figure 1, and a retracted position as illustrated in Figures 2 and 3. A spring 30 is used to bias the rod 16 and needle assembly 20 proximally to the retracted position. It will also be noted with reference to Figure 3 that the needle assembly 20 can be provided with shoulder stops 31 which are separated by a width greater than the internal diameter of the shaft 14. These shoulder stops 31 prevent the distal tip 22 of the needle assembly 20 from entering the shaft 14 in the retracted position.

The bifurcated portions 24 and 26 are also moveable between two positions. In a first position, the bifurcated portions 24 and 26 are separated radially to enlarge the slot 28. In a second position, the bifurcated portions 24 and 26 are brought into close proximately by the tube, closing the slot 28. Preferably, these bifurcated portions 24 and 26 are biased to the separated state as illustrated in Figure 1 so the slot 28 automatically opens when the needle assembly 20 is in the extended position.

Operation of the suture device 10 is illustrated in Figures 4-9. Initially, the device 10 in a relaxed, stored state will have the needle assembly 20 biased to the retracted position as illustrated in Figure 4. In order to initially load the device 10 with a suture 32, the needle assembly must be moved to the extended position as illustrated in Figure 5. This is accomplished by pushing the thumb slide 18 distally to force the actuating rod 16 and the needle assembly 20 in the distal direction. When the needle assembly 20 clears the distal end of hollow shaft 14, the bifurcated portions 24 and 26 are free to expand laterally thereby automatically enlarging the slot 28 as illustrated in Figure 5. With the slot 28 enlarged, the suture 32 can now be passed through the slot as illustrated if Figure 6. Releasing the thumb slide 18 permits the spring 30 to move the actuating rod 16 and needle assembly 20 proximally to the retracted position. As the needle assembly is drawn into the hollow shaft 14, the restricted radial dimension forces the bifurcated portions 24 and 26 to move toward each other thereby closing the slot 28 and fully engaging the suture 32. In this retracted state, illustrated in Figure 7, the device is now loaded and prepared for disposition through the patient's tissue, which is designated by the reference numeral 34 in Figure 8. The actuating rod 16 can be biased to the retracted position so that the device in the relaxed state automatically orients itself for use in crossing the tissue 34 as illustrated in Figure 8.

With the needle assembly 20 and the suture 32 extending through the tissue 34, it is now desirable to release the suture from the device 10. This is accomplished by again pushing the thumb slide distally to move the needle assembly from the retracted position to the extended position. As before, the bias of the bifurcated portions 24 and 26 will permit the needle assembly 20 to enlarge the slot 28 thereby facilitating release of the suture 32 from the device 10. This is illustrated in Figure 9. As a final step, the device 10 can be removed from the tissue 34 leaving the suture 32 operatively disposed across the tissue 34.

Notwithstanding the specific features, it will be noted that the needle assembly 20 includes a sharp point which facilitates passage through the tissue 34 even when the assembly 20 is in the retracted state. The bifurcated portions 24 and 26 function as a gripping means which automatically opens to facilitate loading the suture 32, but which is capable of being closed with a force sufficient to retain the suture in the slot 28 as the device 10 is pushed through the tissue 34. The hollow shaft 14 in this case functions primarily as a vehicle for moving the bifurcated portions 24 and 26 between the open state and the closed state. As illustrated the hollow shaft 14 is shown with a straight configuration; but it can also be provided with many different curved configurations to enhance usage in various anatomically restricted areas. It will be apparent to those skilled in the art that this function can otherwise be accomplished by many different structures.

As illustrated Figure 10, elements of structure similar to those previously discussed are designated with the same reference numeral followed by the lower case letter "a." Thus, this structure includes the actuating rod 16a, needle assembly 20a and distal tip 22a. In this case however, the bifurcated portions 24 and 26 function as a backing arm 36 and gathering arm 38, respectively. The gathering arm 38 can be integral with the backing arm 36 at the distal end of the slot 28a, but preferably is separated from the backing arm 36 at the proximal end of the slot 28a. At this location, the gathering arm 38a is cut from the backing arm 36a to form a passage 40, which allows the needle to be more flexible as it is passed thru curves. The passage 40 can also facilitate drawing the suture into the slot 28a if the passage 40 is exposed distal of the hollow shaft 14a during deployment.

A similar arrangement is illustrated in Figures 11 - 14 wherein elements of structure similar to those previously discussed are designated with the same reference numeral followed by the lower case letter "b." In this case, the hollow shaft 14b receives the actuating rod 16b in the manner previously discussed. This device also includes the backing arm 36b as well as the gathering arm 38b. However, in this case, the gathering arm 38b is integral with the backing arm 36b at the proximal end of the slot 28b. The gathering arm 38b is separated from the backing arm 36b at the distal end where a passage 42 permits the suture to enter the slot 28b. This passage 42 is cut along the gathering arm 38b so that a small portion of the arm 44 remains with the distal tip 22b. This enables the needle assembly 20b to retain the suture without damage when the needle assembly 20b is fully retracted. The gathering arm 38b can be shaped in a variety of ways. In the illustrated example, three bends 46, 48 and 50 create a wide passage 42 to facilitate capture of the suture.

In Figure 12, the needle assembly 20 has been slightly retracted into the hollow shaft 14b and the bend 46 has been generally straightened. The angle of the bend 50 has created a lip that gathers the suture toward the backing arm 36b as the bend 46 is straightened by the hollow shaft 14b.

In Figure 13, the needle assembly 20b is even further retracted. This has caused the bend 48 to straighten bringing the gathering arm 38b into contact with the backing arm 36b while still maintaining the passage 42 for the suture. When fully retracted as illustrated in Figure 14, the short portion 44 of the gathering arm 38b is received in the hollow shaft 14 leaving a small opening 52 to retain the suture.

A further possibility is illustrated in Figure 15 where elements of structure similar to those previously discussed are designated with the same reference numeral followed by the lower case letter "c." The backing arm 36c also defines the slot 28c with the gathering arm 38c. The gathering arm 38c is also cut to form the passage 42c. However, the cut forming this passage 42c forms an oblique angle with the length of the needle assembly 20c so that a stop 54 is formed at the distal end of the gathering arm 38c. This stop 54 is formed with a proximal tang 56 on the gathering arm 38c, which interferes with a distal tang 54 on the short portion 44. It is the purpose of this stop 54 to prevent the gathering arm 38c from contacting the backing arm 36c and thereby restricting free movement of a captured suture.

Another structure is illustrated in Figures 16, 16A and 16B where elements of structure similar to those previously discussed are designated with the same reference numeral followed by the lower case letter "d". The horizontal slot 28d is formed between the bifurcated portions 36d and 38d. With only the single horizontal slot 28d, this structure has a cross-section such as that illustrated in Figure 16a.

Similarly, a vertical slot 60 can be added, leaving the needle assembly 20d with four arms which are designated by the reference numerals 36d', 36d", 38d' and 38d" in Figure 16B. This particular configuration of the needle assembly 20d allows manipulation through drastically bent tubes. Of course, any number of multiple arms could facilitate this advantage. A taper 62 can be formed at the proximal ends of the arms 36d and 38d. This tapered profile keeps the walls of the needle assembly 20d relatively consistent and flexible to minimize stress concentrations.

Figure 17 illustrates a further example wherein a helical slot 64 is cut around the circumference of a cylindrical needle 66. In this case the helical slot 64 is adapted to receive the suture and to hide the suture within the needle 66 as it penetrates the tissue. The helical slot 64 may have a depth greater than zero but preferably not greater than 0.75 times the radius of the needle 66. Figures 18 - 22 illustrate an exemplary method for using the needle assembly 20 and particularly the needle assembly 20b illustrated in Figure 11.

In Figure 18, the needle assembly 20b has been retracted and the hollow shaft 14b passed through the tissue 34b. The suture 32b is shown on the far side of the tissue 34b. With this disposition of the suturing device 10b, the thumb slide 18b can now be moved distally to deploy the bifurcated needle assembly 20b as illustrated in Figure 19. In this position, the suture 32b can be introduced through the distal passage 42 and into the slot 28b. As the thumb slide 18b is drawn proximally, the needle assembly 20b is retracted into the hollow shaft 14b in the matter discussed with reference to Figure 14.

With the suture captured in the opening 52, as illustrated in Figure 20, the suture device can be withdrawn from the tissue 34b pulling the suture 32b through the needle hole left in the tissue 34b. This step in the process is illustrated in Figure 21. Finally, the thumb slide 18b can again be moved distally to deploy the needle assembly 20b. This will produce a wide separation of the backing arm 36b and gathering arm 38b resulting in release of the suture 32b from the suture device 10b. In this manner, the suture 32b can be threaded through the tissue 34b.

A further example is illustrated in Figure 23 where the needle assembly 20 is shown with a unique shape that enhances performance. In this case, the needle assembly 20 includes a backing arm 70 and a gathering arm 72. The gathering arm 72 is curved so that an opening is provided with minimal needle deployment to facilitate capture of the suture. This feature enhances use of the needle assembly 20 where the suture must be engaged in small spaces and the needle cannot be fully deployed.

In Figure 24, a similar structure is illustrated where the backing arm 70 is also curved. In this manner, the needle assembly 20 can be provided with a shape that is closer to that of the hollow shaft 14 (Figure 1). This is representative of a concept wherein the hollow shaft can have many different curved configurations, each adapted for a particular anatomical area or indication; and the associated needle assembly including the backing arm 70 and gathering arm 72 can be matched to the configuration of the shaft.

Figures 25 and 26 show two variations where the needle assembly 20 includes a thick needle section 74 that is proximal of a pointed end 76. The thick needle section 74 defines a portion of the suture slot 67 that, as previously noted, can have a variety of configurations. Proximal of the thick section 74, a diameter can be made smaller so that a thinner section 78 is created. This thin section 78 makes it easier for the needle to be moved through the curves of the hollow shaft 14.

In Figure 27, a notch 81 is created in the distal end of the hollow tube 63. This notch 81, in connection with a needle tip 64, creates a suture slot 83 which holds the suture. With the notch 81 buried in the hollow tube 63, the needle tip 64 can be made shorter so that a curved device can have a lower profile adapting it for use in a smaller diameter cannula.

Figures 28 and 29 illustrate two positions of a needle tip 64 relative to a hollow tube 65. In these two positions, the size of the suture slot 66 can differ. The suture slot 66 in Figure 28 is sufficiently large to enable the suture to slide or slip easily relative to the hollow tube 65. By comparison, the suture slot 66 in Figure 29 is relatively small because the needle tip 64 has been retracted slightly into the hollow tube 65. In this second position, the needle tip 64 is retracted to reduce the size of the suture slot 66 and hold or lock the suture in place against the hollow tube 65. A variety of mechanisms can be used to provide various detented positions between the first position illustrated in Figure 28 and the second position illustrated in Figure 29. A mechanism could also be provided to require a force of progressive magnitude in order to move between the slipping position of Figure 28 and the locking position of Figure 29.

In some cases it is advantageous to provide the hollow shaft 14 with a drastically bent configuration. Under these circumstances, a needle assembly 20 formed from a metal material such a Nitinol, can be made to slide more easily within the drastically bent tube if it is made thinner at those locations where it is to be bent during deployment or retraction. The distal end of the needle assembly 20 can be kept larger so that the diameter of the needle tip is sufficiently thick to withstand any bending stresses. The proximal end need not be made thicker since it functions primarily as a push/pull rod in compression/tension during deployment and retraction.

Figure 30 shows an example of a tapered needle that can be provided in a multitude of configurations. Beginning generally at the distal end, the needle 90 can be provided with a distal taper 81. This taper can start at any location between a point just proximal of the needle point to a location just proximal of the slot. In the latter location, the starting position tends to maximize the amount of material defining the slot. Depending on the strength required, a proximal taper 83 can also be provided back to the diameter of the needle point. This proximal taper 83 would be of particular advantage where a thicker shaft might be required for strength purposes.

As illustrated in Figure 31, many of the foregoing can benefit from a radius 92 provided in the distal end of the slot. Such a radius 92 will insure that a sliding suture is less likely to be frayed on a sharp edge.

In Figure 32, an opening 94 is provided between two tangs 96 and 98, which also define the needle slot. This opening 94 is preferably defined at a location that is more distal along the slot so that it can be exposed when the needle is only slightly deployed from the needle tube. Thus, even with a slight deployment of the needle, a suture can be slid through the opening 94 and into the slot. This can be of particular importance where the suture is very close to where the needle exits the tissue. Under these circumstances, it would be more difficult to capture the suture if the needle had to be fully deployed. Notwithstanding the preferred distal location of the opening 94 relative to the slot, it is desirable that the opening 94 be positioned sufficiently proximally that a basin 96 is formed at the proximal end of the slot, as illustrated in Figure 33.

Figure 34 illustrates a hollow tube housing 101 provided with a taper 103 at its distal end. This taper 103 will tend to minimize the insertion forces required for the device.

A handle assembly 110 is illustrated in Figure 35 as a preferred example of the handle 12 discussed with reference to Figure 1. In the manner previously discussed, the handle assembly 110 is adapted to receive the needle assembly 20, including the hollow shaft 14 and the rod 16. In addition, the needle assembly 20 is provided with a hub 112 having an enlargement 114 and a cylindrical extension 116 disposed proximally of the enlargement 74. The handle assembly 110 includes a frame 118, and the thumb slide 18 that can be moved by a thumb of the user. In this manner, the thumb slide 18 can be moved between a rearward position, to the left in Figure 35, and a forward position, illustrated to the right in Figure 35.

The thumb slide 18 is part of a unit that moves in combination with a slider 121 and a needle latch 123. The needle latch 123 is rotatable on the slider 121 about a pivot pin 125, and includes a leaf spring 127. A tooth 130 is also carried by the slider 121 and cooperates with a slot 132 and ratchet 134 in the frame 118.

A locking lever 136 is pivotal on the frame 118 at a pivot pin 138. This enables the locking lever 136 to move as shown by an arrow 141 between an unlocking position and a locking position illustrated in Figure 35.

A lever latch 143 is biased by a spring 145 to lock the locking lever 136 in the locking position illustrated in Figure 35. Movement of the lever latch 143 longitudinally along an arrow 144 and against the bias of the coil spring 145 frees the locking lever 136 to move from the illustrated locking position to the unlocking position under the bias of a coil spring 147.

When the locking lever 136 is in the unlocking position, a hub lock 150 is removed from proximity with the hub 112 of the needle assembly 20. This enables the cylindrical extension 116 of the needle assembly 20 to be inserted into and removed from a channel 152 at the distal end of the handle assembly 110. With the hub 112 inserted into the channel 152, the locking lever 136 can be moved to its locking position where the hub lock 150 engages a slot 154 in the cylindrical extension 116. The lever latch 143 automatically retains the locking lever 136 in the locking position illustrated in Figure 35.

In order to initially prepare for operation of the suture device 10, the removable needle assembly 20 must be mounted in the handle assembly 110. For purposes of discussion, it will be assumed that the thumb slide 18 is pushed forward into the distal position illustrated in Figure 35.

As an initial step in the mounting process, the locking lever 136 is moved to the unlocked position, downwardly along the arrow 141 in Figure 35. This is accomplished by moving the lever latch 143 rearwardly or proximally to the left in Figure 35. This movement, against the bias of the coil spring 145, will cause the lever latch 143 to disengage the locking lever 136, which will then automatically drop to the unlocking position under the bias of the coil spring 147.

With the hub lock 150 withdrawn from the slot 154, the cylindrical extension 152 of the needle assembly 20 can be moved into the channel 152 along with the needle rod 16. With the needle assembly 20 inserted and the enlargement 114 butted against the handle assembly 110, a notch 155 at the proximal end of the needle rod 16 will engage the needle latch 123. At this point, the locking lever 136 can be closed against the bias of the spring 147 causing the hub lock 150 to engage the slot 154 in the cylindrical extension 152. The closing of the locking lever 136 also provides a positive force through the leaf spring, which maintains the needle latch 123 in the notch 155. As the locking lever 136 moves upwardly to the locking position, the coil spring 145 will bias the lever latch 143 to engage, lock, and retain the locking lever 136 in the locking position. In this operating position, movement of the thumb slide 18 relative to the frame 118 will cause the needle rod 16 to move relative to the hub 114 of the needle assembly 20.

The foregoing procedure for mounting the needle assembly 20 into the handle assembly 110 was begun with the assumption that the thumb slide 18 would be in the forward, distal position. If this is not the case, the needle latch 123 will not initially engage the proximal end of the needle rod 16. Even under these circumstances, the mounting operation can be completed in the foregoing manner so that the hub 114 is retained by the locking lever 136, which is in turn locked by the lever latch 143. With this step completed, the thumb slide 18 can be slid forward in the distal direction to enable the needle latch 123 to engage the notch 155 at the proximal end of the rod 16. This engagement and locking of the rod 16 by the needle latch 123 will be accommodated by deflection of the leaf spring 127. In accordance with either of these processes, the needle assembly 20 can be coupled to the handle assembly 110 and prepared for use by the surgeon.

In operation, the suture 32 can be positioned between the bifurcated portions 24 and 26 of the needle when the needle is deployed. At this point the needle assembly 20 can be fully deployed by moving the thumb slide 10 and the rod 16 distally. In this position of the needle assembly 20, the suture 32 can be loaded, unloaded, picked up, or dropped off as required in different stages of operation.

With the suture 32 thus loaded in the needle assembly 20, the surgeon can pull the thumb slide 18 proximally, rearwardly until the tooth 130 on the slider 121 engages the slot 132 on the frame 118. In this position, the suture 32 is captured within the needle but is still free to slide laterally in the slot of the needle. Note that the springiness of the thumb slide 18 forces the tooth 130 to remain in the slot 132 where it is held in contact with the frame 78.

If it becomes desirable to further engage the suture 32 and eliminate its ability to slide within the needle slot, the thumb slide 18 can be depressed and further retracted causing the tooth 130 to leave the slot 132 and move further rearwardly along the ratchet 134. This slight movement will cause the rod 16 of the needle assembly 20 to move further rearwardly drawing the needle assembly 20 slightly further into the hollow shaft 14. In this position, the suture 32 is not only captured within the needle assembly 20, but is sandwiched and locked between the needle assembly 20 and the hollow shaft 14. During operation of the device 10, the thumb slide 18 is not intended to move further rearwardly.

As it becomes of interest in different stages of operation, the suture 32 can be disengaged by moving the thumb slide 18 from this rearward-most position, forwardly and distally. If the thumb slide is not depressed, the tooth 130 will fall back into the slot 132 where the suture is captured but nevertheless free to slide within the needle eye. In this position, the suture 32 can be removed. By depressing the thumb slide 18 the tooth 130 can be removed from the slot 132 permitting the thumb slide 18 to be moved further in the distal direction. This distal movement will open the bifurcated portions 24 and 26 of the needle assembly 20 and fully releasing the suture 32.

Once the fully operative procedure is completed, the needle assembly 20 can be removed from the handle assembly 110 by reversing the mounting steps previously discussed.

A preferred structure for releasably engaging the needle rod 16 with the needle latch 123 is illustrated in the progressive views of Figures 36A-36D. In these views it is shown that the notch 155 can be formed with a ramp 156 that is machined into the proximal end of the needle rod 16. This ramp 156 allows the needle rod 16 to be pushed into the handle assembly 110 when the enlargement 114 of the hub 112 butts against the handle assembly 110, the proximal end of the needle rod 16 can ride up over a ramp 158 on the needle latch 123 as illustrated in Figure 36B. When fully seated as illustrated Figure 36C, this locking mechanism keeps the needle assembly attached to the handle assembly until the ramp 158 is moved laterally permitting the ramp 156 and associated needle rod 16 to be moved distally.

Apparatus for restricting the range of needle movement between its retracted position and its forward position is illustrated in Figures 37A and 37B. A pin 161 is provided for disposition in a transverse slot 163 within the hub 112. The rod 16 of the needle assembly 20 is also provided with a channel 165 of predetermined length. In this sub-assembly, the rod 16 is initially positioned to extend longitudinally through the hub 112. When the channel 165 intersects the slot 163, the pin 161 can be inserted longitudinally through the slot 163 in the hub 112 and transversely through the channel 165 in the rod 16. This sub-assembly is illustrated in Figure 37B. The restricted movement provided by this sub-assembly is needed so that the needle is always in a position to be loaded into the handle. With the accommodation of this restricted movement, the needle cannot be jammed in the handle assembly 110.

Another unique needle configuration is illustrated the prospective views of Figures 38 and 39. The distal slot is similar to that previously discussed, but the distal tip is bifurcated by a cut 167 that extends into the slot. In operation, deployment of the needle causes the bifurcated portions 169 and 170 to split, as illustrated in Figure 39. Of course there are many variations of this bifurcated configuration. Two such variations are illustrated in Figures 40 and 41. By way of comparison, it will be noted that the cut 167 in Figure 38 is disposed along the axis of the needle tip so that the tip portions are symmetrical. In the views of Figures 40 and 41, the cut 167 does not produce symmetrical tip portions. Although the cut is planar in Figure 41 and in Figure 38, it is nevertheless offset from the axis of the tip in Figure 40. In Figure 41, the cut 167 is non-planar.

Figures 42-47 relate to alternatives that include an actuator assembly that offers a rotational control feature. Here, the needle assembly may be deployed from the distal end of the hollow shaft by manipulating the actuating rod at the proximal end of the hollow shaft using a suitable thumb actuator assembly. Using this same base design, the ability is added such that when the needle assembly is deployed, its arms can also be rotated to get into a better position to grab the suture. The needle assembly can then be rotated back to the original position, if desired, and drawn back in the hollow shaft for withdrawal.

Figs. 42-44 show the distal end of a further possible suture device that can retract and deploy the needle assembly and can rotate the needle assembly to different desired orientations after deployment to aid in the grasping of suture. In particular, Figures 42-44 show sample positions of rotation after a needle assembly 220 having two arms 226, 228 has been deployed from the hollow shaft 214. In Figure 42, the needle assembly 220 is in the standard straight position. In Figure 43, the needle assembly 220 has been rotated about 45°, as suggested by the arrow, and in Figure 44, the needle assembly 220 has been rotated about 135°. This rotation can also occur with the needle assembly only partially deployed.

The rotational action is controlled by providing the user with a suitable control mechanism at the proximal end of the hollow shaft 214 containing the needle assembly 220. There are a variety of possible exemplary methods to rotate the needle assembly.

Figure 45 shows one example of a suture device 210 that includes this rotational enhancement. In particular, Figure 45 is a perspective view of a first suture device 210 that uses a thumb wheel 218 to deploy and rotate the needle assembly 220 as shown in Figures 42-44.

As shown, a handle 212 is attached to hollow shaft 214. Running the length of the shaft 214 is an actuating rod 216 which has its proximal end attached to the thumb wheel 218, and which terminates in split arms 226, 228 at its distal end. The thumb wheel 218 can be used to deploy and retract the split arms 226, 228 relative to the hollow shaft 214 with the reciprocating motion suggested by arrow 204. At any time, the thumb wheel 218 can also be rotated as suggested by arrow 205, which rotation translates along the actuating rod 216 and moves the split arms 226, 228 of the needle assembly 220 in a circular motion suggested by arrow 206.

Figures 46a and 46b show a handle 212 having a thumb lever 318 moveable within a lever channel 319. Figure 46b is a blown-up view of the top of the lever channel 319. The lever 318 can deploy and retract the split arms 226, 228 from the hollow shaft 214 when moved in a back-and-forth direction represented by arrow 304. The lever 318 can also move in a left-and-right direction represented by arrow 305. As best shown in Figure 46b, the lever 318 can move farther and farther in the left-and-right direction as the lever 318 is moved more distally within lever channel 319. The side-to-side motion 305 translates to the split arms 226, 228 and creates a substantially rotational movement as described by arrow 306. As the split arms 226, 228 are retracted by pulling the lever 318 proximally, the side-to-side motion 305 permitted within lever channel 319 becomes lessened until the proximal slot 311 is reached, at which point the side-to-side movement 305 is near zero. The taper described by the lever channel 319 allows more side-to-side movement 305 when the lever 318 is deployed distally so that the split arms 226, 228 can rotate more 306 as they are deployed further. Conversely, as the lever 318 is pulled proximally, the side-to-side movement 305 is restricted to help prevent the split arms 226, 228 from being overstressed in the near closed positions. Within the movement defined by the proximal slot 311 there may be one or more detents or over-ridable stops that position and hold the split arms 226, 228 at a defined position relative to the hollow shaft 214. Figures 47a and 47b are similar to Figures 46a and 46b in that they show a handle 412 having a thumb lever 418 moveable within a lever channel 419.

Here, however, the lever channel 419 defines the movement of the lever 418. There are many ways this can be done but, in this example, the lever 418 is allowed to deploy and retract the split arms 226, 228 by being moved along a central slot 419a. At the distal end, a cross slot 419b allows side-to-side motion of the thumb lever 419. The lever channel 419 may also include additional slots for locking the split arms 226, 228 in a defined location relative to the hollow shaft 214. The first slot is a suture sliding slot 419c and the other is a suture locking slot 419d. When the lever 418 is positioned in the suture sliding slot 419c, a small gap is left between the hook of the split arm 226 and the hollow shaft 214 to allow free sliding of captured suture. When the lever 418 is positioned in the suture locking slot 419d, the gap is lessened so that the captured suture can not move freely.

## Claims

1. A surgical suturing device (410), including
a needle assembly (220) having a needle (226, 228) movable in a needle housing (214) between a free suture state and a captured suture state;
a handle assembly (412) including a longitudinal handle housing sized and configured to releasably receive the needle assembly (220);
a thumb actuator assembly (418) releasably coupled to the needle and movable longitudinally on the handle housing between a distal position and a proximal position and between a first lateral position and a second lateral position;
the distal position of the thumb actuator assembly (418) being associated with the needle in the free suture state;
the proximal position of the thumb actuator assembly (418) being associated with the needle in the captured suture state;
the first lateral position of the thumb actuator assembly (418) being associated with the needle in a first angular position; and
the second lateral position of the thumb actuator assembly (418) being associated with the needle in a second angular position;
wherein the thumb actuator assembly (418) comprises a thumb lever (418) that moves axially along the surgical suturing device's axis and that moves side-to-side about the surgical suturing device's axis.

2. The surgical suturing device (410) recited in Claim 1 wherein the thumb lever assembly (418) further comprises a lever channel (419) that constrains the movement of the thumb lever (418).

3. The surgical suturing device (410) recited in Claim 2 wherein the lever channel (419) is geometrically configured to permit more side-to-side movement of the thumb lever (418) when the thumb lever (418) is positioned distally within the lever channel (419) than when the lever (418) is positioned proximally within the lever channel (419).

4. The surgical suturing device (410) recited in Claim 2 wherein the lever channel (419) comprises a central slot (419a) that permits axial motion of the thumb lever (418) and a cross slot (419b) at a distal end of the central slot (419a) that allows side-to-side motion of the thumb lever (418).

5. The surgical suturing device (410) recited in Claim 4 wherein the lever channel (419) further comprises a slot (419c, 419d) for locking the needle assembly (220) in a defined location relative to a distal end of the handle assembly (412).

6. The surgical suturing device (410) recited in Claim 5 wherein the lever channel (419) comprises a slot (419c) for locking the needle assembly (220) in a defined location associated with the needle in a captured and unlocked suture state to allow free sliding of captured suture.

7. The surgical suturing device (410) recited in Claim 5 or Claim 6 wherein the lever channel (419) comprises a slot (419d) for locking the needle assembly (220) in a defined location associated with the needle in a captured and locked suture state in which captured suture cannot move freely.

## Patentansprüche

1. Chirurgische Nähvorrichtung (410), umfassend
eine Nadelanordnung (220) mit einer Nadel (226, 228), die in einem Nadelgehäuse (214) zwischen einem nicht eingefädelten Zustand und einem eingefädelten Zustand bewegt werden kann;
eine Griffanordnung (412), die ein längliches Griffgehäuse umfasst, das derart bemessen und ausgelegt ist, die Nadelanordnung (220) lösbar aufzunehmen;
eine Daumenbetätigungsanordnung (418), die lösbar mit der Nadel gekoppelt ist und in Längsrichtung an dem Griffgehäuse zwischen einer distalen Position und einer proximalen Position und zwischen einer ersten seitlichen Position und einer zweiten seitlichen Position bewegt werden kann;
wobei die distale Position der Daumenbetätigungsanordnung (418) mit der Nadel in dem freien Nahtzustand verknüpft ist;
wobei die proximale Position der Daumenbetätigungsanordnung (418) mit der Nadel im eingefädelten Zustand verknüpft ist;
die erste seitliche Position der Daumenbetätigungsanordnung (418) mit der Nadel in einer ersten winkligen Position verknüpft ist; und
die zweite seitliche Position der Daumenbetätigungsanordnung (418) mit der Nadel in einer zweiten winkligen Position verknüpft ist;
wobei die Daumenbetätigungsanordnung (418) einen Daumenhebel (418) umfasst, der sich axial entlang der Achse der chirurgischen Nähvorrichtung bewegt und der sich entlang der Achse der chirurgischen Nähvorrichtung von einer Seite zur anderen Seite bewegt.

2. Chirurgische Nähvorrichtung (410) nach Anspruch 1, wobei die Daumenhebelanordnung (418) ferner einen Hebelkanal (419) umfasst, der die Bewegung des Daumenhebels (418) beschränkt.

3. Chirurgische Nähvorrichtung (410) nach Anspruch 2, wobei der Hebelkanal (419) geometrisch dazu ausgelegt ist, mehr Bewegung des Daumenhebels (418) von einer Seite zur anderen Seite zu erlauben, wenn der Daumenhebel (418) distal in dem Hebelkanal (419) positioniert ist, als wenn der Hebel (418) proximal in dem Hebelkanal (419) positioniert ist.

4. Chirurgische Nähvorrichtung (410) nach Anspruch 2, wobei der Hebelkanal (419) einen zentralen Schlitz (419a) umfasst, der die axiale Bewegung des Daumenhebels (418) erlaubt, und einen Querschlitz (419b) an einem distalen Ende des zentralen Schlitzes (419a), der die Bewegung des Daumenhebels (418) von einer Seite zur anderen Seite erlaubt.

5. Chirurgische Nähvorrichtung (410) nach Anspruch 4, wobei der Hebelkanal (419) ferner einen Schlitz (419c, 419d) zum Arretieren der Nadelanordnung (220) in einer definierten Position bezogen auf ein distales Ende der Griffanordnung (412) umfasst.

6. Chirurgische Nähvorrichtung (410) nach Anspruch 5, wobei der Hebelkanal (419) einen Schlitz (419c) zum Arretieren der Nadelanordnung (220) in einer definierten Position umfasst, die mit der Nadel in einem eingefädelten und nicht arretierten Zustand verknüpft ist, um ein freies Gleiten des eingefädelten Fadens zu erlauben.

7. Chirurgische Nähvorrichtung (410) nach Anspruch 5 oder Anspruch 6, wobei der Hebelkanal (419) einen Schlitz (419d) zum Arretieren der Nadelanordnung (220) in einer definierten Position umfasst, die mit der Nadel in einem eingefädelten und arretierten Zustand verknüpft ist, in dem sich der eingefädelte Faden nicht frei bewegen kann.

## Revendications

1. Dispositif de suture chirurgical (410), comprenant
un ensemble formant aiguille (220) comportant une aiguille (226, 228) pouvant être déplacée dans un logement d'aiguille (214) entre un état de suture libre et un état de suture capturé ;
un ensemble formant poignée (412) comprenant un boîtier de poignée longitudinal dimensionné et conçu pour recevoir de manière libérable l'ensemble formant aiguille (220) ;
un ensemble formant actionneur de pouce (418) accouplé de manière libérable à l'aiguille et mobile longitudinalement sur le boîtier de poignée entre une position distale et une position proximale et entre une première position latérale et une deuxième position latérale ;
la position distale de l'ensemble formant actionneur de pouce (418) étant associée à l'aiguille dans l'état de suture libre ;
la position proximale de l'ensemble formant actionneur de pouce (418) étant associée à l'aiguille dans l'état de suture capturé ;
la première position latérale de l'ensemble formant actionneur de pouce (418) étant associée à l'aiguille dans une première position angulaire ; et
la deuxième position latérale de l'ensemble formant actionneur de pouce (418) étant associée à l'aiguille dans une deuxième position angulaire ;
l'ensemble formant actionneur de pouce (418) comprenant un levier de pouce (418) qui se déplace axialement le long de l'axe du dispositif de suture chirurgical et qui se déplace latéralement sur l'axe du dispositif de suture chirurgical.

2. Dispositif de suture chirurgical (410) selon la revendication 1, l'ensemble formant levier de pouce (418) comprenant en outre un canal de levier (419) qui limite le mouvement du levier de pouce (418).

3. Dispositif de suture chirurgical (410) selon la revendication 2, le canal de levier (419) étant conçu géométriquement pour permettre davantage de mouvement latéral du levier de pouce (418) lorsque le levier de pouce (418) est positionné de manière distale à l'intérieur du canal de levier (419) que lorsque le levier (418) est positionné de manière proximale à l'intérieur du canal de levier (419).

4. Dispositif de suture chirurgical (410) selon la revendication 2, le canal de levier (419) comprenant une fente centrale (419a) permettant un mouvement axial du levier de pouce (418) et une fente transversale (419b) à une extrémité distale de la fente centrale (419a) permettant le mouvement latéral du levier de pouce (418).

5. Dispositif de suture chirurgical (410) selon la revendication 4, le canal de levier (419) comprenant en outre une fente (419c, 419d) destinée à verrouiller l'ensemble formant aiguille (220) dans un emplacement défini par rapport à une extrémité distale de l'ensemble formant poignée (412).

6. Dispositif de suture chirurgical (410) selon la revendication 5, le canal de levier (419) comprenant une fente (419c) destinée à verrouiller l'ensemble formant aiguille (220) dans un emplacement défini associé à l'aiguille dans un état de suture capturé et déverrouillé pour permettre le coulissement libre de la suture capturée.

7. Dispositif de suture chirurgical (410) selon la revendication 5 ou la revendication 6, le canal de levier (419) comprenant une fente (419d) destinée à verrouiller l'ensemble formant aiguille (220) dans un emplacement défini associé à l'aiguille dans un état de suture capturé et verrouillé dans lequel la suture capturée ne peut pas bouger librement.
